# EUROPÄISCHE PATENTSCHRIFT

(11) **EP 3 029 052 B1**
(45) Veröffentlichungstag und Bekanntmachung des Hinweises auf die Patenterteilung: **28.02.2018**
(21) Anmeldenummer: 14196192.0
(22) Anmeldetag: 04.12.2014
(51) Int. Cl.: B01J 31/18, C07F 9/6574, C07B 41/06, C07C 45/50

(54) **9-Anthrol-monophosphit Ester als Liganden für Hydroformylierungs-Katalysatoren**
9-Anthrol-monophosphit Esters as Ligands for Hydroformylation Catalysts
Esters monophosphites de 9-Anthrol comme ligands de catalysateurs d'hydroformylation

(43) Veröffentlichungstag der Anmeldung: 08.06.2016
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Geilen, Frank, 45721 Haltern am See (DE); Fridag, Dirk, 45721 Haltern am See (DE); Hess, Dieter, 45770 Marl (DE); Selent, Detlef, 18059 Rostock (DE); Börner, Armin, 18059 Rostock (DE)

(56) Entgegenhaltungen:
- WO-A2-2004/076464
- US-A1- 2006 100 455
- TAKASHI HIRATA ET AL: "DFT Study of Chiral-Phosphoric-Acid-Catalyzed Enantioselective Friedel-Crafts Reaction of Indole with Nitroalkene: Bifunctionality and Substituent Effect of Phosphoric Acid", CHEMISTRY - AN ASIAN JOURNAL, Bd. 6, Nr. 2, 22. Dezember 2010 (2010-12-22), Seiten 510-516, XP055189726, ISSN: 1861-4728, DOI: 10.1002/asia.201000596

## Beschreibung

Die Erfindung betrifft Monophosphite, die ein Anthrol aufweisen. Des Weiteren deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P(III). Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Aus EP 0 155 508 A1 ist die Verwendung von bisarylensubstituierten Monophosphiten bei der rhodiumkatalysierten Hydroformylierung von sterisch gehinderten Olefinen, z. B. Isobuten, bekannt. Hierbei werden jedoch zum Teil sehr hohe Rhodiumkonzentrationen verwendet (u.a. 250 ppm), was in Anbetracht des derzeitigen Rhodiumpreises für ein großtechnisches Verfahren inakzeptabel ist und verbessert werden muss. Auf Seite 41 oben ist eine Verbindung dargestellt, bei der drei Phenylrest jeweils über eine C-C-Brücke miteinander verknüpft sind, hierbei handelt sich um eine 2,6-Biphenyl-Phenol-Einheit.

In der WO 2004/076464 A2 werden optisch aktive Phosphite und Phosphoramidite als Liganden beschrieben. Des Weiteren wird ein Verfahren zur Herstellung chiraler Verbindungen beschrieben. Hierbei wird eine prochirale Verbindung in Anwesenheit eines chiralen Übergangsmetall-Katalysators umgesetzt, wobei der Katalysator jene optisch aktiven Liganden aufweist.

In US 2006/100455 A1 wird ein Verfahren beschrieben, bei welchem ethylenisch ungesättigte Verbindungen hydrocyaniert werden zu Verbindungen, welche mindestens eine Nitrilfunktion aufweisen. Die Hydrocyanierung wird hierbei durch einen Metall-Ligand-Komplex katalysiert.

In TAKASHI HIRATA ET AL, "DFT Study of Chiral-Phosphoric-Acid-Catalyzed Enantioselective Friedel-Crafts Reaction of Indole with Nitroalkene: Bifunctionality and Substituent Effect of Phosphoric Acid", CHEMISTRY - AN ASIAN JOURNAL, (20101222), Vol. 6, Nr. 2, doi:10.1002/asia.201000596, ISSN 1861-4728, Seiten 510 - 516 werden Berechnungen zu Friedel-Crafts-Reaktionen beschrieben. Zur Aufklärung der hohen Enantioselektivität wurden DFT-Rechnungen durchgeführt.

Der Erfindung lag die Aufgabe zugrunde, Monophosphite bereitzustellen, welche gegenüber den bekannten Monophosphiten vorteilhafte Eigenschaften in der Hydroformylierung aufweisen. Insbesondere bestand die Aufgabe darin, neue Liganden bereitzustellen, deren Einsatz gegenüber strukturverwandten Monophosphiten, welche zusätzlich zu der Biphenoleinheit ebenfalls weitere aromatische Systeme aufweisen, zu einer verbesserten Ausbeute führen. Die verbesserte Ausbeute sollte bei zumindest einem Olefin realisiert werden.

Wünschenswerter Weise zusätzlich auch bei einer Metallkonzentration (beispielsweise Rhodium) von weniger als 100 ppm.

Die Aufgabe wird gelöst durch eine Verbindung nach Anspruch 1.

Verbindung, welche eine der allgemeinen Strukturen **I** oder **II** aufweist: 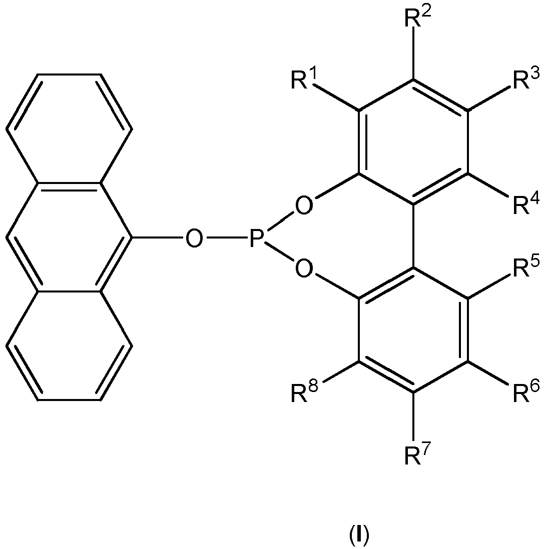 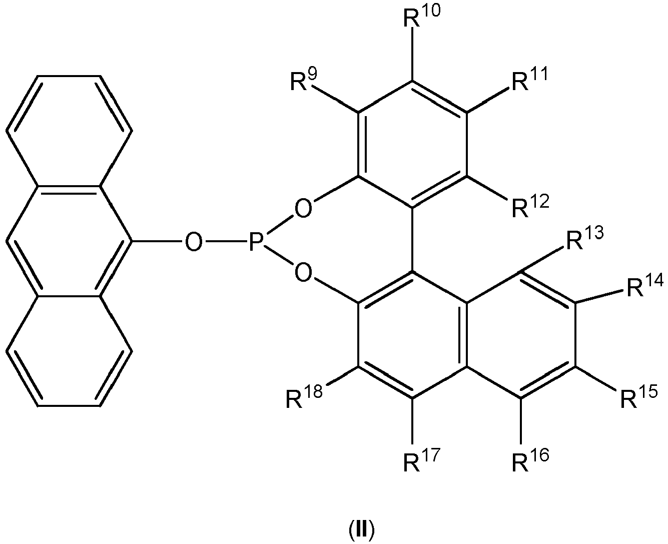 wobei
R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
   substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
   substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, - COOH, -OH, -SO₃H; -SO₃Na, -NO₂, -CN, -NH₂, -N[(C₁-C₁₂)-Alkyl]₂.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf.

Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte - (C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl, -O-(C₆-C₂₀)-Aryl.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl.

In einer Ausführungsform sind R¹, R², R³, R⁴, R⁵, R⁶, R⁷, R⁸ ausgewählt aus: -H, -(C₁-C₁₂)-Alkyl, -O-(C₁-C₁₂)-Alkyl.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl.

In einer Ausführungsform weist die Verbindung eine der folgenden Strukturen (**1**) bis (**10**) auf:

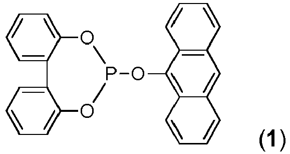

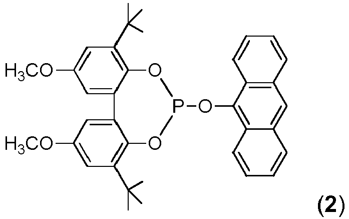

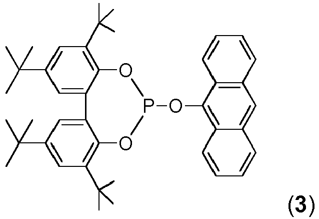

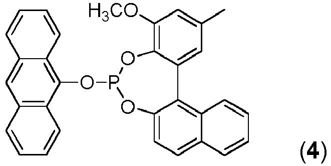

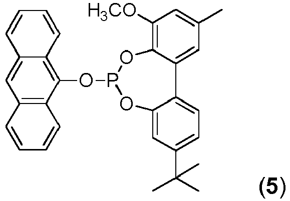

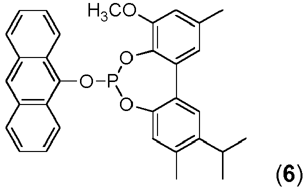

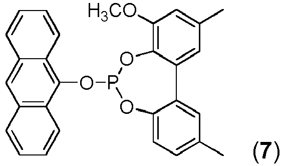

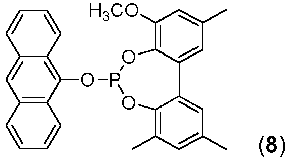

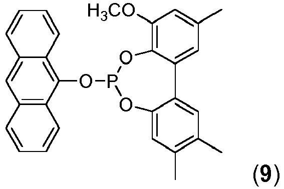

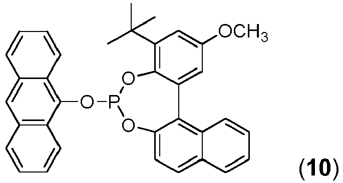

In einer Ausführungsform weist die Verbindung die allgemeinen Strukturen **I** auf.

In einer Ausführungsform weist die Verbindung die allgemeinen Strukturen **II** auf.

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 200 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Biphenole

Die Synthese der Biphenole erfolgt analog zu DE102013203865 und DE102013203867.

### Synthese der Chlorophosphite

Die Synthese der Mono- und Dichlorophosphite wie (Anthracen-9-yloxy)dichlorophosphin, 2,6-Diphenylphenoxydichlorphosphin oder 6-Chlorodibenzo[*d*,*f*][1,3,2]dioxaphosphepin ist dem Fachmann bekannt und erfolgt nach bekannter Weise. Chlorophosphite lassen sich durch Zusatz von Phosphortrichlorit in Gegenwart einer Base herstellen. Für nähere Informationen siehe auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Synthese der Monophosphite

### 6-(Anthracen-9-yloxy)dibenzo[d,f][1,3,2]dioxaphosphepin.

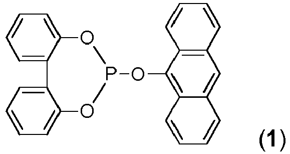

Eine gerührte Lösung von Anthracen-9-ol (0,389 g; 2,00 mmol) in Toluol (9 ml) wurde mit Triethylamin (1,858 g; 18,36 mmol) versetzt und die erhaltene Mischung tropfenweise bei 0 °C zu einer Lösung von 6-Chlorodibenzo[*d*,*f*][1,3,2]dioxaphosphepin (0,501 g; 2,00 mmol) in Toluol (9 ml) gegeben. Die Reaktionsmischung wurde über Nacht gerührt und filtriert. Das Filtrat wurde im Vakuum bis zur Trockne eingeengt. Der erhaltene Feststoff wurde für 2 h bei 40 °C getrocknet und dann aus heißem Dichlormethan (3 ml) umkristallisiert. Ausbeute: 0,206 g (0,504 mmol; 25 %).

Elementaranalyse (berechnet für C₂₆H₁₇O₃P = 408,37 g/ mol) C 76,59 (76,47); H 4,14 (4,20); P 7,64 (7,58) %.
³¹P-NMR (CD₂Cl₂): 148,4 ppm.
¹H-NMR (CD₂Cl₂): 7,37-7,72 (m, 12 H); 8,10 (m, 2 H); 8,40 (s, 1 H); 8,56 (m, 2 H) ppm. ¹³C-NMR (CD₂Cl₂): 122,6; 122,9; 123,7; 125,0; 126,1; 126,2; 126,4; 128,6; 129,8; 130,6; 131,5; 132,5; 143,4 (d, *J*_{CP}= 6,0 Hz); 149,4 (d, *J*_{CP}= 5,8 Hz) ppm.
ESI-TOF/HRMS: m/e 409,09945 (M+H)⁺.

### 6-(Anthracen-9-yloxy)-4,8-di-tert-butyl-2,10-dimethoxydibenzo[d,f][1,3,2]dioxaphosphepin.

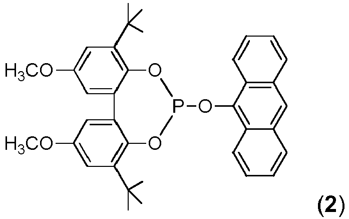

Eine gerührte Lösung von Anthracen-9-ol (0,388 g; 2 mmol) in Toluol (9 ml) wurde mit Triethylamin (1,854 g; 18,33 mmol) versetzt und die erhaltene Mischung tropfenweise bei 0 °C zu einer Lösung von 4,8-Di-*tert*-butyl-6-chloro-2,10-dimethoxydibenzo[*d*,*f*][1,3,2] dioxaphosphepin (0,845 g; 2 mmol) in Toluol (8 ml) gegeben. Die Reaktionsmischung wurde über Nacht gerührt und filtriert. Das Filtrat wird im Vakuum bis zur Trockne eingeengt. Der erhaltene Rückstand wurde zweimal mit Hexan (je 4 ml) verrührt und filtriert, und dann im Vakuum getrocknet. Ausbeute: 0,206 g (0,355 mmol; 18 %).

Elementaranalyse (berechnet für C₃₆H₃₇O₅P = 580,63 g/ mol) C 74,28 (74,46); H 6,62 (6,42); P 5,39 (5,33) %.
³¹P-NMR (CD₂Cl₂): 140,4 ppm.
¹H-NMR (CD₂Cl₂): 1,39 (s, 18 H); 3,92 (s, 6 H); 6,93 (d, ⁵*J*_{HH}= 3,1 Hz, 2 H, Hₐᵣₒₘ); 7,14 (d, ⁵*J*_{HH}= 3,1 Hz, 2 H, Hₐᵣₒₘ); 7,51 (m, 4 H, Hₐᵣₒₘ); 8,05 (m, 2 H, Hₐᵣₒₘ); 8,34 (s, 1 H, Hₐᵣₒₘ); 8,41 (m, 2 H, Hₐᵣₒₘ) ppm.
¹³C-NMR (CD₂Cl₂): 31,0; 35,7; 56,0; 113,5; 114,9; 123,2; 123,7 (d, *J*_{CP}= 5,4 Hz); 124,8; 125,9; 126,0; 128,4; 132,6; 134,2 (d, *J*_{CP}= 3,9 Hz); 141,7 (d, *J*_{CP}= 6,0 Hz); 143,4; 143,5; 156,6
ppm.
ESI-TOF/HRMS: m/e 581,24490 (M+H)⁺.

### 6-(Anthracen-9-yloxy)-2,4,8,10-tetra-tert-butyldibenzo[d,f][1,3,2]dioxaphosphepin.

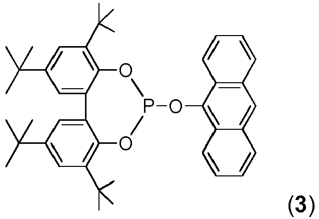

Eine gerührte Suspension von Anthracen-9-ol (0,281 g; 1,45 mmol) in Toluol (7 ml) wurde mit Triethylamin (1,344 g; 13,28 mmol) versetzt und die erhaltene Mischung tropfenweise bei 0 °C zu einer Lösung von 2,4,8,10-Tetra-*tert*-butyl-6-chlorodibenzo[*d*,*f*][1,3,2]dioxaphosphepin (0,724 g; 1,52 mmol) in Toluol (6 ml) gegeben. Die Reaktionsmischung wurde über Nacht gerührt und filtriert. Das Filtrat wurde im Vakuum bis zur Trockne eingeengt und der erhaltene Rückstand aus Hexan (4 ml) umkristallisiert. Ausbeute: 0,559 g (0,883 mmol; 61 %).

Elementaranalyse (berechnet für C₄₂H₄₉O₃P = 632,78 g/ mol) C 79,83 (79,71); H 7,61 (7,81); P 5,01 (4,89) %.
³¹P-NMR (CD₂Cl₂): 140,7 ppm.
¹H-NMR (CD₂Cl₂): 1,41 (s, 18 H, C(C*H*₃)₃); 1,50 (s, 18 H, C(C*H*₃)₃); 7,40 (d, ⁵*J*_{HH}= 2,4 Hz, 2 H, Hₐᵣₒₘ); 7,43-7,56 (m, 4 H, Hₐᵣₒₘ); 7,60 (d, ⁵*J*_{HH}= 2,4 Hz, 2 H, Hₐᵣₒₘ); 8,05 (m, 2 H, Hₐᵣₒₘ); 8,34 (s, 1 H, Hₐᵣₒₘ); 8,38 (m, 2 H, Hₐᵣₒₘ) ppm.
¹³C-NMR (CD₂Cl₂): 31,2; 31,2; 31,8; 35,1; 35,7; 123,1; 123,7 (d, *J*_{CP}= 5,3 Hz); 124,8; 125,0; 125,8; 126,0; 127,2; 128,4; 132,6; 133,3 (d, *J*_{CP}= 3,7 Hz); 141,0; 143,5; 145,8 (d, *J*_{CP}= 6,2 Hz); 147,7 ppm.
ESI-TOF/HRMS: m/e 633,34934 (M+H)⁺.

### 6-(Anthracen-9-yloxy)-4-methoxy-2-methylbenzo[d]naphtho[1,2-f][1,3,2]dioxaphosphepin.

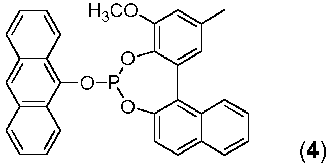

Eine Lösung von 1-(2-Hydroxy-3-methoxy-5-methylphenyl)naphthalen-2-ol (0,448 g; 1,598 mmol) in THF (8 ml) wurde mit Pyridin (0,284 g; 3,596 mmol) versetzt. Dann wurde eine Lösung von (Anthracen-9-yloxy)dichlorophosphin (0,472 g; 1,598 mmol) in THF (6 ml) tropfenweise bei 0°C zugegeben. Die Reaktionsmischung wurde über Nacht gerührt, filtriert und das Filtrat im Vakuum bis zur Trockne eingeengt. Der erhaltene Rückstand wurde aus heißem Dichlormethan (13 ml) umkristallisiert. Ausbeute: 0,302 g (0,601 mmol; 38 %).

Elementaranalyse (berechnet für C₃₂H₂₃O₄P = 502,50 g/ mol) C 76,38 (76,49); H 4,68 (4,61); P 6,15 (6,16) %.
³¹P-NMR (THF-d₈): 147,3; 152,7 ppm.
¹H-NMR (THF-d₈): 29-2,31 (2s, 3 H); 3,77-3,87 (2s, 3 H); 6,92 (m, 1 H, Hₐᵣₒₘ); 7,01 (m, 1 H, Hₐᵣₒₘ); 7,34-7,46 (m, 7 H, Hₐᵣₒₘ); 7,47-7,58 (m, 1 H, Hₐᵣₒₘ); 7,81-7,95 (m, 4 H, Hₐᵣₒₘ); 8,11 (m, 1 H, Hₐᵣₒₘ); 8,25 (m, 1 H, Hₐᵣₒₘ); 8,47 (m, 1 H, Hₐᵣₒₘ); 8,74 (m, 1 H, Hₐᵣₒₘ) ppm.
¹³C-NMR (THF-d₈): 20,6; 20,7; 55,2; 55,5; 112,4; 112,8; 121,4; 121,6; 122,6; 123,0; 123,0; 123,8; 124,0; 124,6; 124,6; 124,9; 125,1; 125,5;125,5; 125,6; 125,7; 125,7; 126,2; 126,2; 126,6; 126,7; 127,9; 128,0; 128,4; 128,4; 128,5; 128,5; 129,5; 130,0; 130,2 (d, *J*_{CP}= 4,3 Hz); 132,0; 132,3; 132,5; 134,0; 134,8; 135,7; 137,6; 143,2 (d, *J*_{CP}= 7,7 Hz); 143,5 (d, *J*_{CP}= 8,3 Hz); 145,8 (d, *J*_{CP}= 2,8 Hz); 147,2 (d, *J*_{CP}= 6,7 Hz); 152,2; 152,5 (d, *J*_{CP}= 2,6 Hz) ppm.
ESI-TOF/HRMS: m/e 503,14057 (M+H)⁺.

### 6-(Anthracen-9-yloxy)-9-(tert-butyl)-4-methoxy-2-methyldibenzo[d,f][1,3,2]dioxaphosphepin.

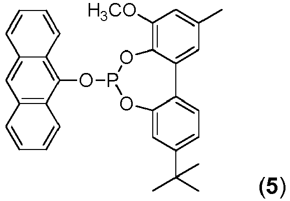

Eine gerührte Suspension von 4'-(*tert*-Butyl)-3-methoxy-5-methyl-[1,1'-biphenyl]-2,2'-diol (0,703 g; 2,46 mmol) in Toluol (7 ml) wurde mit Triethylamin (2,277 g; 22,50 mmol) versetzt und diese Mischung tropfenweise bei 0 °C zu einer Lösung von (Anthracen-9-yloxy)dichlorophosphin (0,725 g; 2,46 mmol) in Toluol (13 ml) gegeben. Die Reaktionsmischung wurde über Nacht gerührt und dann filtriert. Das Filtrat wurde im Vakuum bis zur Trockne eingeengt und der erhaltene Rückstand aus Toluol (2 ml) umkristallisiert. Ausbeute: 0,320 g (0,630 mmol; 26 %).

Elementaranalyse (berechneet für C₃₂H₂₉O₄P = 508,55 g/ mol) C 75,48 (75,58); H 5,86 (5,75); P 6,03 (6,09) %.
³¹P-NMR (CD₂Cl₂): 148,4 ppm.
¹H-NMR (CD₂Cl₂): 44 (s, 9 H); 2,49 (m, 3 H); 4,00 (s, 3 H); 6,94-7,04 (m, 2 H, Hₐᵣₒₘ); 7,46 (m, 2 H, Hₐᵣₒₘ); 7,56-7,69 (m, 5 H, Hₐᵣₒₘ); 8,10 (m, 2 H, Hₐᵣₒₘ); 8,39 (m, 1 H, Hₐᵣₒₘ); 8,78 (m, 2 H, Hₐᵣₒₘ) ppm.
¹³C-NMR (CD₂Cl₂): 21,7; 31,3; 31,4; 35,1; 56,5; 113,0; 119,5; 121,9; 123,2; 123,3; 123,5; 125,2 (d, *J*_{CP}= 5,4 Hz); 126,3 (d, *J*_{CP}= 9,5 Hz); 128,5; 129,9; 132,0 (d, *J*_{CP}= 3,0 Hz); 132,6; 136,1; 136,2 (d, *J*_{CP}= 6,0 Hz); 143,9 (d, *J*_{CP}= 6,7 Hz); 149,3 (d, *J*_{CP}= 5,7 Hz); 152,1; 153,8 ppm.
ESI-TOF/HRMS: m/e 509,18744 (M+H)⁺.

### 6-(Anthracen-9-yloxy)-2-isopropyl-8-methoxy-3,10-dimethyldibenzo[d,f][1,3,2]dioxaphosphepin.

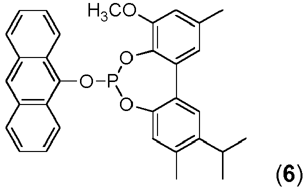

Eine Lösung von 5'-Isopropyl-3-methoxy-4',5-dimethyl-[1,1'-biphenyl]-2,2'-diol (0,359 g; 1,252 mmol) in Toluol (7 ml) wurde mit Triethylamin (1,161 g; 11,473 mmol) versetzt, auf 0 °C gekühlt und zu dieser Mischung eine Lösung von (Anthracen-9-yloxy)dichlorophosphin (0,370 g; 1,252 mmol) in Toluol (9 ml) tropfenweise zugegeben. Die Reaktionsmischung wurde über Nacht gerührt und dann filtriert. Das Filtrat wurde im Vakuum bis zur Trockne eingeengt. Ausbeute: 0,594 g (1,168 mmol; 93 %).

Elementaranalyse (berechnet für C₃₂H₂₉O₄P = 508,55 g/ mol) C 75,46 (75,58); H 5,90 (5,75); P 6,09 (6,09) %.
³¹P-NMR (CD₂Cl₂): 148,5 ppm.
¹H-NMR (CD₂Cl₂): 38 (d, ²*J*_{HH} = 6,8 Hz, 3 H); 1,39 (d, ²*J*_{CP} = 6,8 Hz, 3 H); 2,46 (s, 3 H); 2,52 (s, 3 H); 3,22-3,32 (m, 1 H); 3,99 (s, 3 H); 6,95 (m, 1 H, Hₐᵣₒₘ); 7,07 (m, 1 H, Hₐᵣₒₘ); 7,20 (m, 1 H, Hₐᵣₒₘ); 7,52 (s, 1 H, Hₐᵣₒₘ); 7,57-7,68 (m, 4 H, Hₐᵣₒₘ); 8,10 (m, 2 H, Hₐᵣₒₘ); 8,39 (m, 1 H, Hₐᵣₒₘ); 8,79 (m, 2 H, Hₐᵣₒₘ) ppm.
¹³C-NMR (CD₂Cl₂): 19,3; 21,7; 23,6; 23,6; 29,5; 56,5; 113,0; 121,9; 123,4; 123,5; 123,8; 125,2; (d, *J*_{CP}= 3,3 Hz); 125,7; 126,3 (d, *J*_{CP}= 3,3 Hz); 126,7; 128,6 (d, *J*_{CP}= 8,7 Hz); 129,4; 132,5 (d, *J*_{CP}= 3,6 Hz); 132,6; 136,1; 136,2 (d, *J*_{CP}= 6,2 Hz); 137,5; 138,4; 143,9 (d, *J*_{CP}= 6,5 Hz); 144,8; 146,9 (d, *J*_{CP}= 5,9 Hz); 152,1 ppm.
ESI-TOF/HRMS: m/e 509,18763 (M+H)⁺.

### 6-(Anthracen-9-yloxy)-4-methoxy-2,10-dimethyldibenzo[d,f][1,3,2]dioxaphosphepin.

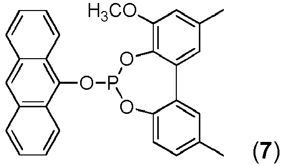

Eine gerührte Lösung von 3-Methoxy-5,5'-dimethyl-[1,1'-biphenyl]-2,2'-diol (0,655 g; 2,68 mmol) in Toluol (16 ml) wurde mit Triethylamin (2,487 g; 24,58 mmol) versetzt und auf 0 °C gekühlt. Zu dieser Mischung wurde eine Lösung von (Anthracen-9-yloxy)dichlorophosphin (0,792 g; 2,683 mmol) in Toluol (18 ml) tropfenweise zugegeben. Die Reaktionsmischung wurde über Nacht gerührt und dann filtriert. Das Filtrat wurde im Vakuum bis zur Trockne eingeengt und der Rückstand 3 h bei 50°C / 0,1 mbar getrocknet. Ausbeute: 1,020 g (2,187 mmol; 82 %).

Elementaranalyse (berechnet für C₂₉H₂₃O₄P = 466,47 g/ mol) C 74,58 (74,67); H 5,19 (4,97); P 6,78 (6,64) %.
³¹P-NMR (CD₂Cl₂): 148,6 ppm.
¹H-NMR (CD₂Cl₂): 2,51 (m, 6 H); 3,99 (s, 3 H); 6,96 (m, 1 H, Hₐᵣₒₘ); 7,06 (m, 1 H, Hₐᵣₒₘ); 7,32-7,37 (m, 2 H, Hₐᵣₒₘ); 7,48 (m, 1 H, Hₐᵣₒₘ); 7,57-7,69 (m, 4 H, Hₐᵣₒₘ); 8,10 (m, 2 H, Hₐᵣₒₘ); 8,39 (m, 1 H, Hₐᵣₒₘ); 8,79 (m, 2 H, Hₐᵣₒₘ) ppm.
¹³C-NMR (CD₂Cl₂): 21,1; 21,7; 56,4; 113,2; 122,0; 122,2; 123,3; 123,5; 125,1 (d, *J*_{CP}= 3,7 Hz); 126,2; 126,3; 128,5; 130,3; 130,9; 131,3 (d, *J*_{CP}= 3,2 Hz); 132,1 (d, *J*_{CP}= 3,3 Hz); 132,5 (d, *J*_{CP}= 2,0 Hz); 135,7; 136,1; 136,3 (d, *J*_{CP}= 5,8 Hz); 143,9 (d, *J*_{CP}= 7,1 Hz); 147,3 (d, *J*_{CP}= 5,4 Hz); 152,0 (d, *J*_{CP}= 2,0 Hz) ppm.
ESI-TOF/HRMS: m/e 467,10093 (M+H)⁺.

### 6-(Anthracen-9-yloxy)-4-methoxy-2,8,10-trimethyldibenzo[d,f][1,3,2]dioxaphosphepin.

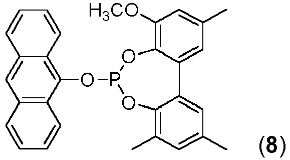

Eine Lösung von 3-Methoxy-3',5,5'-trimethyl-[1,1'-biphenyl]-2,2'-diol (0,409 g; 1,58 mmol) in Toluol (9 ml) wurde mit Triethylamin (2,468 g; 14,51 mmol) versetzt, auf 0 °C gekühlt und zu dieser Mischung eine Lösung von (Anthracen-9-yloxy)dichlorophosphin (0,467 g; 1,584 mmol) in Toluol (11 ml) tropfenweise zugegeben. Die Reaktionsmischung wurde über Nacht gerührt und dann filtriert. Das Filtrat wurde im Vakuum bis zur Trockne eingeengt und der erhaltene Rückstand aus Hexan (17 ml) umkristallisiert. Ausbeute: 0,511 g (1,063 mmol; 67 %).

Elementaranalyse (berechnet für C₃₀H₂₅O₄P = 480,50 g/ mol) C 74,53 (74,99); H 5,53 (5,24); P 6,48 (6,45) %.
³¹P-NMR (CD₂Cl₂): 147,7 ppm.
¹H-NMR (CD₂Cl₂): 2,46-2,50 (m, 9 H); 4,02 (s, 3 H); 6,96 (m, 1 H, Hₐᵣₒₘ); 7,05 (m, 1 H, Hₐᵣₒₘ); 7,20 (m, 1 H, Hₐᵣₒₘ); 7,30 (m, 1 H, Hₐᵣₒₘ); 7,56-7,68 (m, 4 H, Hₐᵣₒₘ); 8,10 (m, 2 H, Hₐᵣₒₘ); 8,40 (m, 1 H, Hₐᵣₒₘ); 8,88 (m, 2 H, Hₐᵣₒₘ) ppm.
¹³C-NMR (CD₂Cl₂): 17,0; 21,1; 21,7; 56,3; 113,0; 122,2; 123,4; 123,5; 125,3 (d, *J*_{CP}= 3,6 Hz); 125,7; 126,3 (d, *J*_{CP}= 5,5 Hz); 128,4; 128,5; 130,9; 131,6 (d, *J*_{CP}= 3,7 Hz); 132,0; 132,0; 132,5; 135,4; 135,8; 136,8 (d, *J*_{CP}= 8,0 Hz); 143,9 (d, *J*_{CP}= 7,4 Hz); 145,3 (d, *J*_{CP}= 4,0 Hz); 151,9 ppm.
ESI-TOF/HRMS: m/e 481,15626 (M+H)⁺.

### 6-(Anthracen-9-yloxy)-8-methoxy-2,3,10-trimethyldibenzo[d,f][1,3,2]dioxaphosphepin.

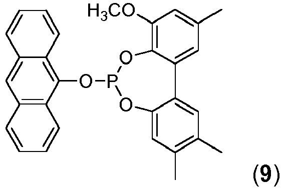

Eine Lösung von 3-Methoxy-4',5,5'-trimethyl-[1,1'-biphenyl]-2,2'-diol (0,518 g; 2,00 mmol) in Toluol (11 ml) wurde mit Triethylamin (1,857 g; 18,35 mmol) versetzt und auf 0 °C gekühlt. Zu dieser Mischung wurde eine Lösung von (Anthracen-9-yloxy)dichlorophosphin (0,591 g; 2,00 mmol) in Toluol (8 ml) tropfenweise zugegeben. Nach Zugabe von weiterem Toluol (30 ml) wurde die Reaktionsmischung über Nacht gerührt. Dann wurde die Mischung filtriert und das Lösungsmittel im Vakuum entfernt. Der erhaltene Rückstand wurde in Toluol (11 ml) aufgenommen, die resultierende Suspension leicht erhitzt und dann filtriert. Das Filtrat wurde im Vakuum bis zur Trockne eingeeengt und der erhaltene Feststoff bei 50°C / 0.1 mbar getrocknet. Ausbeute: 0,489 g (1,018 mmol; 51 %).

Elementaranalyse (berechnet für C₃₀H₂₅O₄P = 480,50 g/ mol) C 74,77 (74,99); H 5,10 (5,24); P 6,47 (6,45) %.
³¹P-NMR (CD₂Cl₂): 148,2 ppm.
¹H-NMR (CD₂Cl₂): 2,38 (m, 6 H); 2,49 (m, 3 H); 3,98 (s, 3 H); 6,93 (m, 1 H, Hₐᵣₒₘ); 7,03 (m, 1 H, Hₐᵣₒₘ); 7,19 (m, 1 H, Hₐᵣₒₘ); 7,40 (m, 1 H, Hₐᵣₒₘ); 7,56-7,68 (m, 4 H, Hₐᵣₒₘ); 8,09 (m, 2 H, Hₐᵣₒₘ); 8,38 (m, 1 H, Hₐᵣₒₘ); 8,76 (m, 2 H, Hₐᵣₒₘ) ppm.
¹³C-NMR (CD₂Cl₂): 19,4; 19,8; 21,7; 56,4; 112,9; 121,8; 123,3; 123,3; 123,4; 125,1 (d, *J*_{CP}= 3,5 Hz); 125,6 (d, *J*_{CP}= 3,5 Hz); 126,1; 126,2; 128,4; 131,1; 132,1 (d, *J*_{CP}= 3,1 Hz); 132,5; 134,4; 136,0; 136,2 (d, *J*_{CP}= 6,0 Hz); 138,8; 143,9 (d, *J*_{CP}= 6,9 Hz); 147,2 (d, *J*_{CP}= 5,7 Hz); 152,0 ppm.
ESI-TOF/HRMS: m/e 481,15601 (M+H)⁺.

### 6-(Anthracen-9-yloxy)-4-(tert-butyl)-2-methoxybenzo[d]naphtho[1,2-f][1,3,2]dioxaphosphepin.

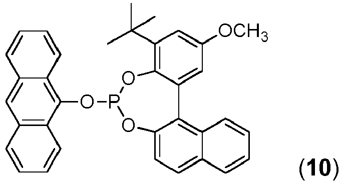

Eine Lösung von 1-(3-(*tert*-Butyl)-2-hydroxy-5-methoxyphenyl)naphthalen-2-ol (0,730 g; 2,26 mmol) in Toluol (14 ml) wurde mit Triethylamin (2,097 g; 20,73 mmol) versetzt und zu dieser Mischung bei 0 °C eine Lösung von (Anthracen-9-yloxy)dichlorophosphin (0,668 g; 2,26 mmol) in Toluol (10 ml) tropfenweise zugegeben. Die Reaktionsmischung wurde über Nacht gerührt und dann filtriert. Das Filtrat wurde im Vakuum bis zur Trockne eingeengt und der erhaltene Rückstand aus Hexan (28 ml) umkristallisiert. Ausbeute: 0,708 g (1,30 mmol; 58 %).

Elementaranalyse (berechnet für C₃₅H₂₉O₄P = 544,58 g/ mol) C 76,96 (77,19); H 5,38 (5,37); P 5,66 (5,69) %.
³¹P-NMR (CD₂Cl₂): 150,0 ppm.
¹H-NMR (CD₂Cl₂): 53 (s, 9 H); 3,88 (s, 3 H); 7,10 (d, *J*_{HH} = 3,1 Hz, 1 H, Hₐᵣₒₘ); 7,18 (d, *J*_{HH} = 3,1 Hz, 1 H, Hₐᵣₒₘ); 7,54-7,64 (m, 6 H, Hₐᵣₒₘ); 7,78 (m, 1 H, Hₐᵣₒₘ); 7,99-8,10 (m, 4 H, Hₐᵣₒₘ); 8,18-8,22 (m, 1 H, Hₐᵣₒₘ); 8,38 (m, 1 H, Hₐᵣₒₘ); 8,62 (m, 2 H, Hₐᵣₒₘ) ppm.
¹³C-NMR (CD₂Cl₂): 31,2; 35,8; 56,1; 115,0; 115,1; 121,7; 123,1; 123,7; 125,0 (d, *J*_{CP}= 3,7 Hz); 125,7; 126,0; 126,2; 126,4; 127,3; 128,6; 128,9; 129,4; 130,1; 130,7 (d, *J*_{CP}= 5,0 Hz); 132,4; 132,5; 133,0; 138,4; 142,1; 143,7 (d, *J*_{CP}= 7,2 Hz); 144,4; 146,8 (d, *J*_{CP}= 5,5 Hz); 156,1 ppm. ESI-TOF/HRMS: m/e 545,18764 (M+H)⁺.

### 6-([1,1':3',1"-Terphenyl]-2'-yloxy)-4,8-di-tert-butyl-2,10-dimethoxydibenzo[d,f][1,3,2]dioxaphosphepin.

### (Vergleichsverbindung)

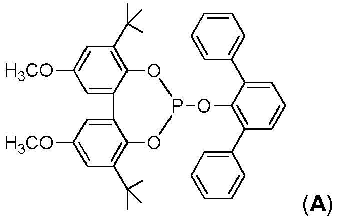

Eine Lösung von 2,6-Diphenylphenol (0,411 g; 1,65 mmol) in Toluol (8 ml) wurde mit Triethylamin (1,529 g; 15,11 mmol) versetzt und die erhaltene Mischung tropfenweise bei 0°C zu einer Lösung von 4,8-Di-*tert*-butyl-6-chloro-2,10-dimethoxydibenzo[*d*,*f*][1,3,2]dioxaphosphepin (0,697 g; 1,65 mmol) in Toluol (6 ml) gegeben. Die Reaktionsmischung wurde über Nacht bei Raumtemperatur und für 5 h bei 70 °C gerührt. Dann wurde die Mischung filtriert und das Filtrat im Vakuum bis zur Trockne eingeengt. Der erhaltene Rückstand wurde aus Hexan (4 ml) umkristallisiert. Ausbeute: 0,417 g (0,659 mmol; 40 %).

Elementaranalyse (berechnet für C₄₀H₄₁O₅P = 632,70 g/ mol) C 75,95 (75,93); H 6,52 (6,53); P 5,01 (5,00) %.
³¹P-NMR (CD₂Cl₂): 140,9 ppm.
¹H-NMR (CD₂Cl₂): 1,37 (s, 18 H); 3,84 (s, 6 H); 6,51 (d, ⁵*J*_{HH}= 3,1 Hz, 2 H, Hₐᵣₒₘ); 6,89-7,95 (m, br, 15 H, Hₐᵣₒₘ) ppm.
¹³C-NMR (CD₂Cl₂): 31,1; 35,5; 55,9; 113,0; 114,5; 125,2; 126,5-131,0 (broad, overlapping signals); 133,8 (d, *J*_{CP}= 4,0 Hz); 141,5 (d, *J*_{CP}= 6,3 Hz); 142,6; 144,8; 156,1 ppm.
ESI-TOF/HRMS: m/e 633,27654 (M+H)⁺.

### 6-([1,1':3',1"-Terphenyl]-2'-yloxy)-2,4,8,10-tetra-tert-butyldibenzo[d,f][1,3,2]dioxaphosphepin.

### (Vergleichsverbindung)

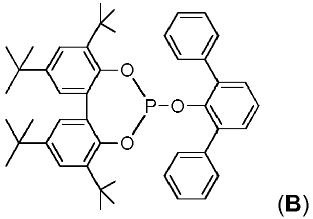

Eine Lösung von 3,3',5,5'-Tetra-*tert*-butyl-[1,1'-biphenyl]-2,2'-diol (0,616 g; 1,50 mmol) in Toluol (13 ml) wurde mit Triethylamin (1,390 g; 13,74 mmol) versetzt und auf 0 °C gekühlt. Zu dieser Mischung wurde eine Lösung von (2,6-Diphenylphenoxy)dichlorophosphin (0,521 g; 1,50 mmol) in Toluol (3 ml) tropfenweise zugegeben. Die Reaktionsmischung wurde über Nacht gerührt und filtriert. Das Filtrat wurde im Vakuum bis zur Trockne eingeengt, der erhaltene Rückstand in Dichlormethan (3 ml) aufgenommen und über Kieselgel filtriert. Das Filtrat wurde im Vakuum eingeengt und der erhaltene Feststoff bei 50°C / 0,1 mbar getrocknet. Ausbeute: 0,955 g (1,394 mmol; 93 %).

Elementaranalyse (berechnet für C₄₆H₅₃O₃P = 684,90 g/ mol) C 80,60 (80,67); H 7,71 (7,80); P 4,36 (4,52) %.
³¹P-NMR (CD₂Cl₂): 138,7 ppm.
¹H-NMR (CD₂Cl₂): 1,29-1,46 (m, br, 36 H, C(C*H*₃)₃); 6,76-7,13 (m, br, 4 H, Hₐᵣₒₘ); 7,30-7,34 (m, 1 H, Hₐᵣₒₘ); 7,34-7,40 (m, 2 H, Hₐᵣₒₘ); 7,40-7,87 (m, br, 10 H, Hₐᵣₒₘ) ppm.
¹³C-NMR (CD₂Cl₂): 31,3; 31,3; 31,7; 34,9; 35,6; 124,6; 125,2; 125,7; 126,7; 128,6; 129,4; 133,0; 137,7; 138,4; 140,5; 143,5; 144,9; 145,5; 146,9; 150,2; zusätzliche breite Signale zwischen 127 und 131 ppm.
ESI-TOF/HRMS: m/e 685,38089 (M+H)⁺.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Als Olefin wurde ein Octengemisch eingesetzt: n-Octene (Oxeno GmbH, Octenisomerengemisch aus 1-Octen: ∼3 %; *cis*+*trans*-2-Octen: ∼49%; *cis*+*trans*-3-Octen: ∼29%; *cis*+*trans*-Octen-4: ∼16%; gerüstisomere Octene: ∼3 % wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse, s.u.) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). Der Autoklav wurde bei einem Gesamtgasdruck (Synthesegas: Linde; H₂ (99,999%): CO (99,997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar bzw. b) 19,5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Katalyseergebnisse

Die Ergebnisse der Katalyseversuche sind in den Tabellen 1 und 2 zusammengefasst.

**Tabelle 1:**

| **Ligand** | **p (bar)** | **T (°C)** | **t (h)** | **[Rh] (ppm)** | **L/Rh** | **Ausb. (%)** |
|---|---|---|---|---|---|---|
| **1*** | 50 | 120 | 4 | 100 | 5 | 98 |
| **2*** | 50 | 120 | 4 | 100 | 5 | 97 |
| **2*** | 50 | 120 | 4 | 60 | 5 | 97 |
| **2*** | 50 | 120 | 4 | 40 | 5 | 96 |
| **3*** | 50 | 120 | 4 | 100 | 5 | 98 |
| **4*** | 50 | 120 | 4 | 100 | 5 | 92 |
| **5*** | 50 | 120 | 4 | 100 | 5 | 100 |
| **6*** | 50 | 120 | 4 | 100 | 5 | 99 |
| **7*** | 50 | 120 | 4 | 100 | 5 | 99 |
| **7*** | 50 | 120 | 4 | 40 | 5 | 100 |
| **7*** | 50 | 120 | 4 | 60 | 5 | 100 |
| **8*** | 50 | 120 | 4 | 100 | 5 | 99 |
| **8*** | 50 | 120 | 4 | 40 | 5 | 99 |
| **8*** | 50 | 120 | 4 | 60 | 5 | 100 |
| **9*** | 50 | 120 | 4 | 100 | 5 | 99 |
| **10*** | 50 | 120 | 4 | 100 | 5 | 98 |

| | | | | | | |
|---|---|---|---|---|---|---|
| * erfindungsgemäße Verbindung Olefin: n-Octene (Oxeno GmbH) Solvens: Toluol Verhältnis Ligand/Rhodium (L/Rh): 5 : 1 | | | | | | |

Wie aus Tabelle 1 ersichtlich ist, konnte mit allen erfindungsgemäßen Verbindungen eine sehr gute Ausbeute erzielt werden. Auch eine Reduzierung der Rhodiumkonzentration von 100 ppm auf 60 ppm und 40 ppm wird durch die hohe Ligandenaktivität kompensiert, sodass weiterhin Ausbeuten von über 90% erreicht werden konnten.

Dies spielt insbesondere bei einem großtechnischen Einsatz eine wichtige Rolle, da man möglichst geringe Mengen des teuren Metalls Rhodium einsetzen möchte und dennoch hohe Ausbeuten an gewünschtem Produkt erhalten will.

**Tabelle 2:**

| **Ligand** | **p (bar)** | **T (°C)** | **t (h)** | **[Rh] (ppm)** | **L/Rh** | **Ausb. (%)** |
|---|---|---|---|---|---|---|
| **3*** | 20 | 120 | 4 | 100 | 5 | 86 |
| **4*** | 20 | 120 | 4 | 100 | 5 | 91 |
| **7*** | 20 | 120 | 4 | 100 | 5 | 98 |
| **8*** | 20 | 120 | 4 | 100 | 5 | 98 |
| **A** | 20 | 120 | 4 | 100 | 5 | 29 |
| **B** | 20 | 120 | 4 | 100 | 5 | 21 |

| | | | | | | |
|---|---|---|---|---|---|---|
| * erfindungsgemäße Verbindung Olefin: n-Octene (Oxeno GmbH) Solvens: Toluol Verhältnis Ligand/Rhodium (L/Rh): 5 : 1 | | | | | | |

Vier der in Tabelle 1 gelisteten Verbindungen wurden auch bei einem geringeren Druck (20 bar) getestet. Alle vier Verbindungen führten auch hier zu sehr guten Ausbeuten.

Mit den erfindungsgemäßen Verbindungen konnte eine deutlich bessere Ausbeute erzielt werden, als mit den Vergleichsverbindungen **(A)** und **(B).**

Anhand der oben beschriebenen Versuche konnte gezeigt werden, dass die gestellte Aufgabe durch die erfindungsgemäßen Verbindungen gelöst wird.

## Patentansprüche

1. Verbindung, welche eine der allgemeinen Strukturen **I** oder **II** aufweist: 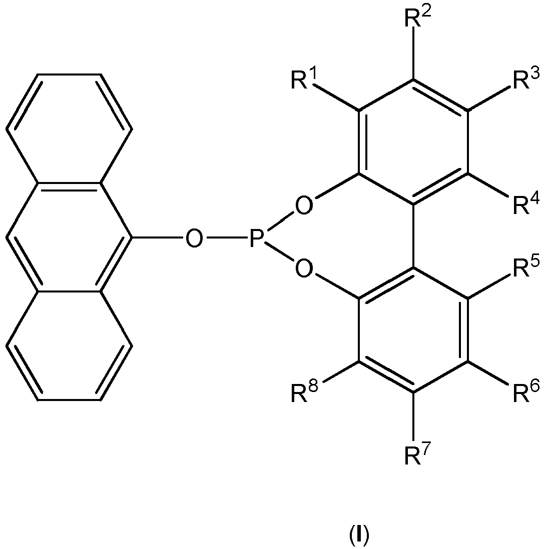 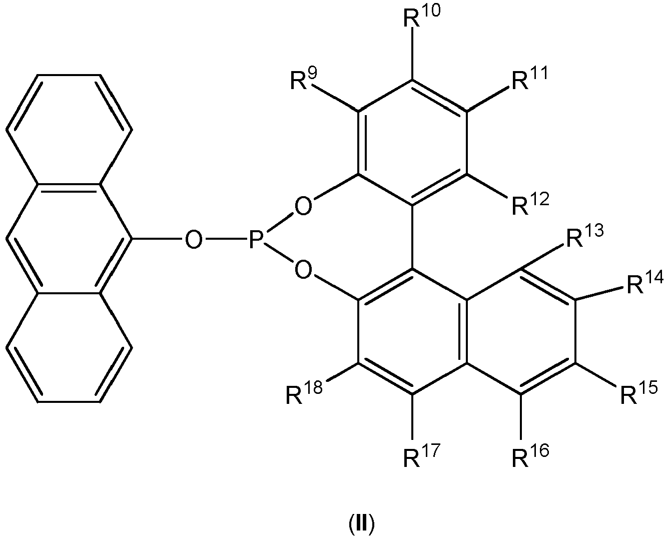 wobei
R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, - COOH, -OH, -SO₃H; -SO₃Na, -NO₂, -CN, -NH₂, -N[(C₁-C₁₂)-Alkyl]₂.

2. Verbindung nach Anspruch 1,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

3. Verbindung nach einem der Ansprüche 1 oder 2,
wobei R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

4. Verbindung nach einem der Ansprüche 1 bis 3,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

5. Verbindung nach einem der Ansprüche 1 bis 4,
wobei R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

6. Verbindung nach einem der Ansprüche 1 bis 5,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl.

7. Verbindung nach einem der Ansprüche 1 bis 6,
wobei R⁹, R¹⁰, R¹¹, R¹², R¹³, R¹⁴, R¹⁵, R¹⁶, R¹⁷, R¹⁸ausgewählt sind aus:
-H, -(C₁-C₁₂)-Alkyl, -O-(C₁-C₁₂)-Alkyl, -O-(C₆-C₂₀)-Aryl.

8. Verbindung nach einem der Ansprüche 1 bis 7,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl.

9. Verbindung nach einem der Ansprüche 1 bis 8,
wobei R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl.

10. Verbindung nach einem der Ansprüche 1 bis 9,
welche eine der folgenden Strukturen **1** bis **10** aufweist:

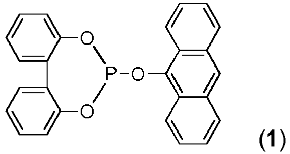

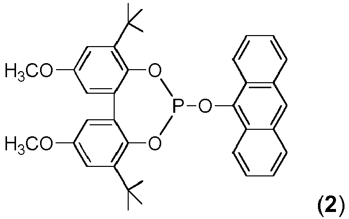

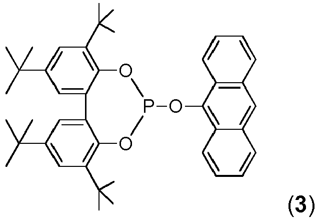

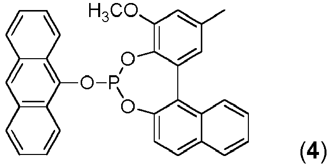

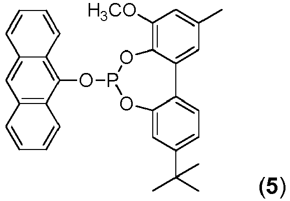

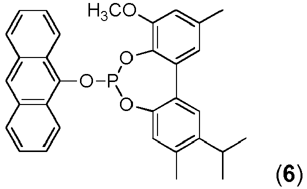

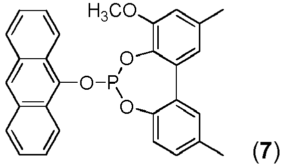

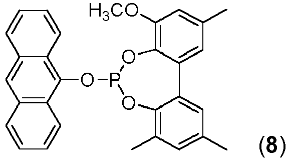

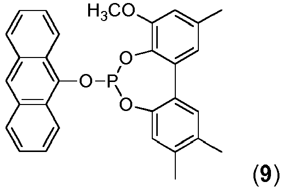

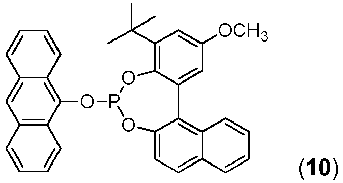

11. Verbindung nach einem der Ansprüche 1 bis 10,
wobei die Verbindung die allgemeinen Strukturen **I** aufweist.

12. Verbindung nach einem der Ansprüche 1 bis 10,
wobei die Verbindung die allgemeinen Strukturen **II** aufweist.

13. Komplex umfassend:
- eine Verbindung nach einem der Ansprüche 1 bis 12,
- ein Metallatom ausgewählt aus: Rh, Ru, Co, Ir.

14. Verwendung einer Verbindung nach einem der Ansprüche 1 bis 12,
zur Katalyse einer Hydroformylierungsreaktion.

15. Verfahren umfassend die Verfahrensschritte:
a) Vorlegen eines Olefins,
b) Zugabe eines Komplexes nach Anspruch 13,
oder einer Verbindung nach einem der Ansprüche 1 bis 12 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having one of the general structures **I** or **II:** 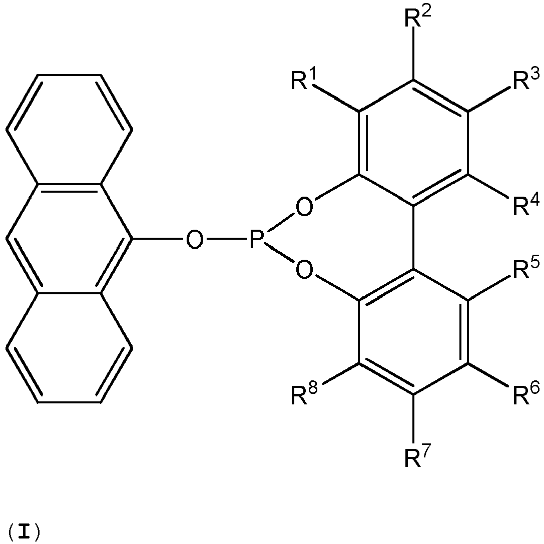 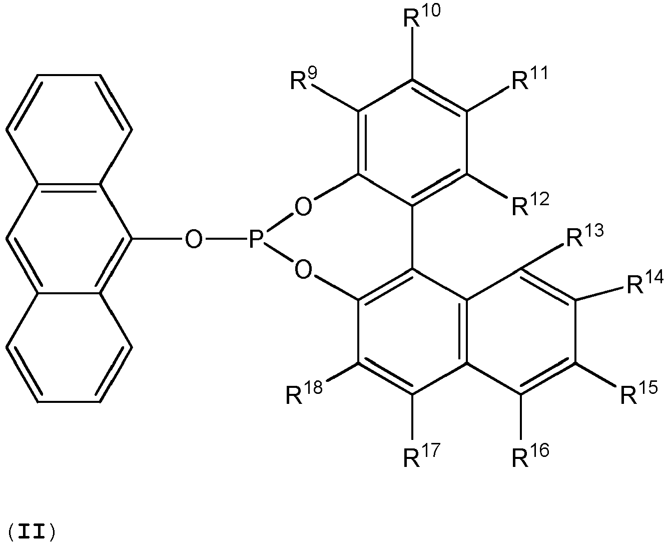 where
R¹, R², R³, R⁴, R⁵, R⁶, R⁷, R⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂;
where the alkyl and aryl groups mentioned may be substituted as follows:
substituted-(C₁-C₁₂)-alkyl groups and substituted-(C₁-C₁₂)-alkoxy groups may have one or more substituents, depending on their chain length; the substituents are each independently selected from-(C₃-C₁₂)-cycloalkyl, - (C₃-C₁₂)-heterocycloalkyl, - (C₆-C₂₀)-aryl, fluorine, chlorine, cyano, formyl, acyl and alkoxycarbonyl;
substituted - (C₆-C₂₀)-aryl groups and -(C₆-C₂₀)-aryl-(C₆-C₂₀)-aryl groups may have one or more substituents, depending on the ring size; these substituents are each independently selected from -H, -(C₁-C₁₂)-alkyl, -O-(C₁-C₁₂)-alkyl, -O-(C₆-C₂₀)-aryl, -(C₆-C₂₀)-aryl, -halogen (such as Cl, F, Br, I), -COO-(C₁-C₁₂)-alkyl, -CONH-(C₁-C₁₂)-alkyl, -(C₆-C₂₀)-aryl-CON[(C₁-C₁₂)-alkyl]₂, -CO-(C₁-C₁₂)-alkyl, -CO-(C₆-C₂₀)-aryl, -COOH, -OH, -SO₃H, -SO₃Na, -NO₂, -CN, -NH₂, -N[(C₁-C₁₂)-alkyl]₂.

2. Compound according to Claim 1,
where R¹, R², R³, R⁴, R⁵, R⁶, R⁷, R⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -CO-(C₁-C₁₂)-alkyl, -CO-(C₆-C₂₀)-aryl, -N[(C₁-C₁₂)-alkyl]₂.

3. Compound according to either of Claims 1 and 2,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -CO-(C₁-C₁₂)-alkyl, -CO-(C₆-C₂₀)-aryl, -N[(C₁-C₁₂)-alkyl]₂.

4. Compound according to any of Claims 1 to 3,
where R¹, R², R³, R⁴, R⁵, R⁶, R⁷, R⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen.

5. Compound according to any of Claims 1 to 4,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen.

6. Compound according to any of Claims 1 to 5,
where R¹, R², R³, R⁴, R⁵, R⁶, R⁷, R⁸ are selected from:
-H, -(C₁-C₁₂)-alkyl, -O-(C₁-C₁₂)-alkyl, -O-(C₆-C₂₀)-aryl.

7. Compound according to any of Claims 1 to 6,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl, -O-(C₆-C₂₀)-aryl.

8. Compound according to any of Claims 1 to 7,
where R¹, R², R³, R⁴, R⁵, R⁶, R⁷, R⁸ are selected from:
-H, -(C₁-C₁₂)-alkyl, -O-(C₁-C₁₂)-alkyl.

9. Compound according to any of Claims 1 to 8,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl.

10. Compound according to any of Claims 1 to 9 having one of the following general structures 1 to 10:

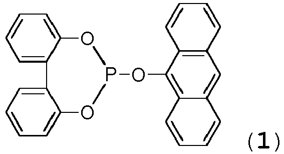

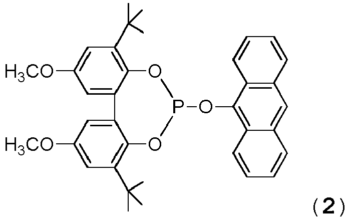

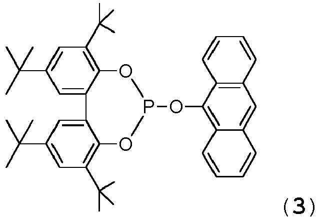

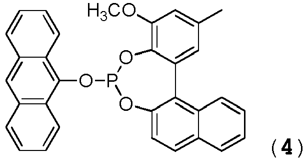

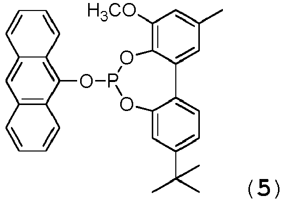

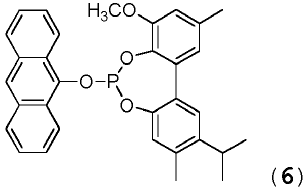

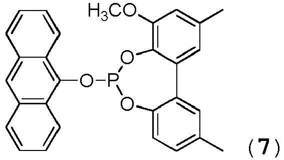

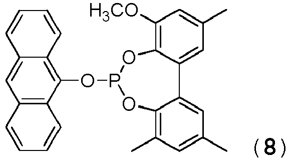

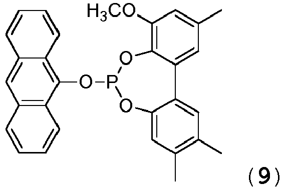

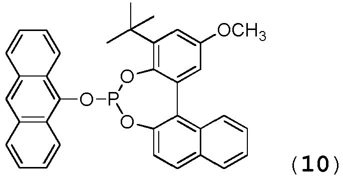

11. Compound according to any of Claims 1 to 10, wherein the compound has the general structures **I.**

12. Compound according to any of Claims 1 to 10, wherein the compound has the general structures **II.**

13. Complex comprising:
- a compound according to any of Claims 1 to 12,
- a metal atom selected from: Rh, Ru, Co, Ir.

14. Use of a compound according to any of Claims 1 to 12,
for catalyzing a hydroformylation reaction.

15. Process comprising the following process steps:
a) initially charging an olefin,
b) adding a complex according to Claim 13,
or a compound according to any of Claims 1 to 12 and a substance having a metal atom selected from: Rh, Ru, Co, Ir.
c) feeding in H₂ and CO,
d) heating the reaction mixture, with conversion of the olefin to an aldehyde.

## Revendications

1. Composé qui présente une des structures générales I ou II : 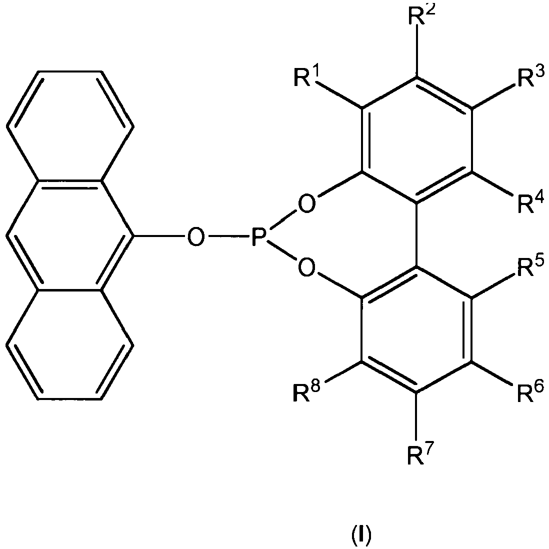 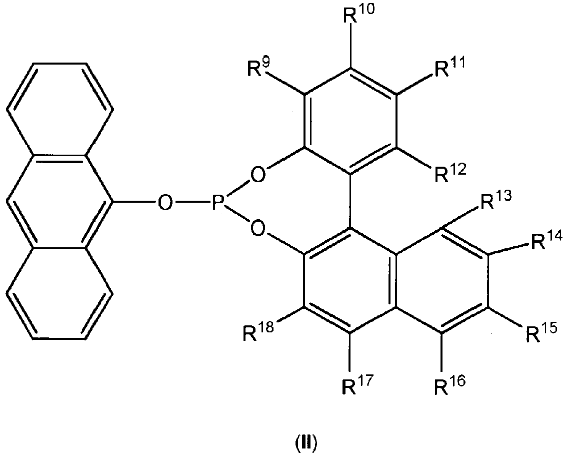 dans lesquelles
R¹, R², R³, R⁴, R⁵, R⁶, R⁷, R⁸ sont choisis parmi :
-H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogéno, -COO-(C₁-C₁₂)-alkyle, -CONH- (C₁-C₁₂)-alkyle, -CO-(C₁-C₁₂)-alkyle, -CO-(C₆-C₂₀)-aryle, -COOH, -OH, -SO₃H, -CN, -NH₂, -N[(C₁-C₁₂)-alkyle]₂ ;
R⁹, R¹⁰, R¹¹, R¹², R¹³, R¹⁴, R¹⁵, R¹⁶, R¹⁷, R¹⁸ sont choisis parmi : -H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogéno, -COO-(C₁-C₁₂)-alkyle, -CONH-(C₁-C₁₂)-alkyle, -CO-(C₁-C₁₂)-alkyle, -CO-(C₆-C₂₀)-aryle, -COOH, -OH, -SO₃H, -NH₂, -N[(C₁-C₁₂)-alkyle]₂ ;
les groupes alkyle et aryle mentionnés pouvant être substitués comme suit :
les groupes -(C₁-C₁₂)-alkyle substitués et les groupes -(C₁-C₁₂)-alcoxy substitués peuvent présenter, en fonction de leur longueur de chaîne, un ou plusieurs substituants ; les substituants sont choisis, indépendamment les uns des autres, parmi -(C₃-C₁₂)-cycloalkyle, -(C₃-C₁₂)-hétérocycloalkyle, -(C₆-C₂₀)-aryle, fluoro, chloro, cyano, formyle, acyle ou alcoxycarbonyle ;
les groupes -(C₆-C₂₀)-aryle substitués et les groupes -(C₆-C₂₀)-aryl-(C₆-C₂₀)-aryle substitués peuvent présenter, en fonction de leur dimension de cycle, un ou plusieurs substituants ; ces substituants sont choisis, indépendamment les uns des autres, parmi -H, -(C₁-C₁₂)-alkyle, -O-(C₁-C₁₂)-alkyle, -O-(C₆-C₂₀)-aryle, -(C₆-C₂₀)-aryle, -halogéno, (tel que Cl, F, Br, I) -COO-(C₁-C₁₂)-alkyle, -CONH-(C₁-C₁₂)-alkyle, -(C₆-C₂₀)-aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, -N[(C₁-C₁₂) -alkyle]₂.

2. Composé selon la revendication 1,
R¹, R², R³, R⁴, R⁵, R⁶, R⁷, R⁸ étant choisis parmi :
-H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogéno, -CO-(C₁-C₁₂)-alkyle, -CO-(C₆-C₂₀)-aryle, -N[(C₁-C₁₂)-alkyle]₂.

3. Composé selon l'une quelconque des revendications 1 ou 2,
R⁹, R¹⁰, R¹¹, R¹², R¹³, R¹⁴, R¹⁵, R¹⁶, R¹⁷, R¹⁸ étant choisis parmi :
-H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogéno, -CO-(C₁-C₁₂)-alkyle, -CO-(C₆-C₂₀)-aryle, -N[(C₁-C₁₂)-alkyle]₂.

4. Composé selon l'une quelconque des revendications 1 à 3,
R¹, R², R³, R⁴, R⁵, R⁶, R⁷, R⁸ étant choisis parmi :
-H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogéno.

5. Composé selon l'une quelconque des revendications 1 à 4,
R⁹, R¹⁰, R¹¹, R¹², R¹³, R¹⁴, R¹⁵, R¹⁶, R¹⁷, R¹⁸ étant choisis parmi :
-H, -(C₁-C₁₂)-alkyle, -O-(C₁-C₁₂)-alkyle, -O-(C₆-C₂₀)-aryle, -(C₆-C₂₀)-aryle, -S-alkyle, -S-aryle, halogéno.

6. Composé selon l'une quelconque des revendications 1 à 5,
R¹, R², R³, R⁴, R⁵, R⁶, R⁷, R⁸ étant choisis parmi :
H, -(C₁-C₁₂)-alkyle, -O-(C₁-C₁₂)-alkyle, -O-(C₆-C₂₀)-aryle.

7. Composé selon l'une quelconque des revendications 1 à 6,
R⁹, R¹⁰, R¹¹, R¹², R¹³, R¹⁴, R¹⁵, R¹⁶, R¹⁷, R¹⁸ étant choisis parmi :
-H, -(C₁-C₁₂)-alkyle, -O-(C₁-C₁₂)-alkyle, -O-(C₆-C₂₀)-aryle.

8. Composé selon l'une quelconque des revendications 1 à 7,
R¹, R², R³, R⁴, R⁵, R⁶, R⁷, R⁸ étant choisis parmi :
-H, -(C₁-C₁₂)-alkyle, -O-(C₁-C₁₂)-alkyle.

9. Composé selon l'une quelconque des revendications 1 à 8,
R⁹, R¹⁰, R¹¹, R¹², R¹³, R¹⁴, R¹⁵, R¹⁶, R¹⁷, R¹⁸ étant choisis parmi :
-H, -(C₁-C₁₂)-alkyle, -O-(C₁-C₁₂)-alkyle.

10. Composé selon l'une quelconque des revendications 1 à 9, qui présente une des structures 1 à 10 suivantes :

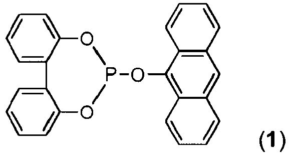

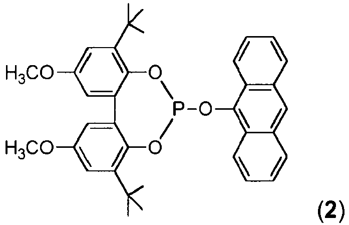

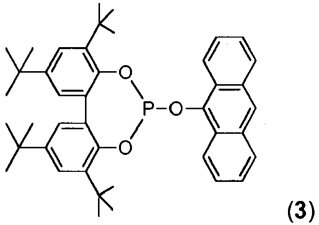

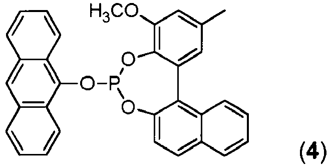

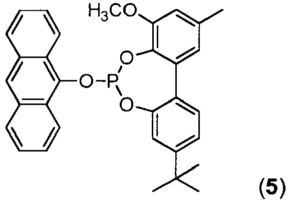

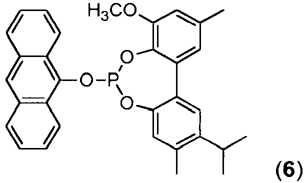

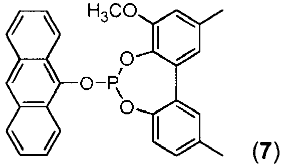

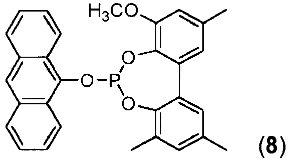

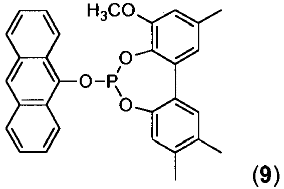

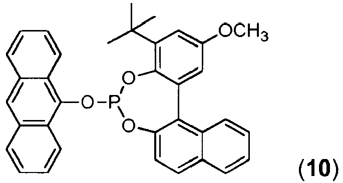

11. Composé selon l'une quelconque des revendications 1 à 10,
le composé présentant les structures générales I.

12. Composé selon l'une quelconque des revendications 1 à 10,
le composé présentant les structures générales II.

13. Complexe, comprenant :
- un composé selon l'une quelconque des revendications 1 à 12,
- un atome métallique choisi parmi : Rh, Ru, Co, Ir.

14. Utilisation d'un composé selon l'une quelconque des revendications 1 à 12
pour la catalyse d'une réaction d'hydroformylation.

15. Procédé comprenant les étapes de procédé :
a) disposition préalable d'une oléfine,
b) addition d'un complexe selon la revendication 13 ou d'un composé selon l'une quelconque des revendications 1 à 12 et d'une substance qui présente un atome métallique choisi parmi : Rh, Ru, Co, Ir,
c) introduction de H₂ et de CO,
d) chauffage du mélange réactionnel, l'oléfine étant transformée en aldéhyde.
